# EUROPÄISCHE PATENTANMELDUNG

(11) **EP 1 891 996 A2**
(43) Veröffentlichungstag der Anmeldung: **27.02.2008**
(21) Anmeldenummer: 07013430.9
(22) Anmeldetag: 10.07.2007
(51) Int. Cl.: A61L 31/14, A61L 31/16

(54) **Verbesserung der Stabilität biodegradierbarer metallischer Stents, Verfahren und Verwendungen**

(30) Priorität: 07.08.2006 DE 102006038235
(71) Anmelder: BIOTRONIK VI Patent AG, 6341 Baar (CH)
(72) Erfinder: Wittchow, Eric Dr., 90403 Nürnberg (DE)
(74) Vertreter: Lindner-Vogt, Karin L.

(57) **Zusammenfassung**

Die vorliegende Erfindung betrifft einen biodegradierbaren metallischen Stent mit verbesserten Stabilitätseigenschaften nach Implantation des Stents, Verfahren zur Herstellung eines solchen stabilisierteren Stents, Verfahren zur Verbesserung der Stabilisierung eines biodegradierbaren metallischen Stents sowie eine Verwendung von gefäßerweiternden Wirkstoffen zur Verbesserung der Stabilität eines biodegradierbaren metallischen Stents.

## Beschreibung

Die vorliegende Erfindung betrifft einen biodegradierbaren metallischen Stent mit verbesserten Stabilitätseigenschaften nach Implantation des Stents, Verfahren zur Herstellung eines solchen stabilisierteren Stents, Verfahren zur Verbesserung der Stabilisierung eines biodegradierbaren metallischen Stents sowie eine Verwendung von gefäßerweiternden Wirkstoffen zur Verbesserung der Stabilität eines biodegradierbaren metallischen Stents.

Stents im Allgemeinen sind endovaskuläre Prothesen bzw. Implantate, die beispielsweise zur Behandlung von Stenosen verwendet werden. Stents sind außerdem bekannt für die Behandlung von Aneurysmen.

Stents weisen grundsätzlich eine Tragstruktur auf, die geeignet ist, die Wand eines Gefäßes in geeigneter Weise abzustützen, um so das Gefäß zu weiten bzw. ein Aneurysma zu überbrücken. Stents werden dazu in einem komprimierten Zustand in das Gefäß eingeführt und dann an dem zu behandelnden Ort aufgeweitet und gegen die Gefäßwand gedrückt. Dieses Aufweiten kann beispielsweise mit Hilfe eines Ballonkatheters erfolgen. Alternativ sind auch selbstexpandierende Stents bekannt. Diese sind beispielsweise aus einem superelastischen Metall, wie Nitinol, aufgebaut.

Stents werden derzeit in zwei Grundtypen eingeteilt, die dauerhaften Stents und die biodegradierbaren Stents. Dauerhafte Stents sind so ausgestaltet, dass sie im Gefäß für einen unbestimmten Zeitraum verbleiben können. Biodegradierbare Stents hingegen werden über einen vorbestimmten Zeitraum hinweg in einem Gefäß abgebaut. Vorzugsweise werden biodegradierbare Stents erst abgebaut, wenn das traumatisierte Gewebe des Gefäßes verheilt ist und somit der Stent nicht weiter im Gefäßlumen verbleiben muss.

Als biodegradierbare Stentmaterialien sind beispielsweise biodegradierbare Metalllegierungen, Polymere oder Verbundwerkstoffe, die eine ausreichende strukturelle Tragfähigkeit aufweisen, um das Gefäßlumen über einen vorbestimmten Zeitraum stützen zu können, bekannt.

Aus WO 2005/102222 ist bekannt, dass metallische Stents möglicherweise Irritationen des sie umgebenden Gefäßgewebes hervorrufen können, aufgrund dessen, dass Metall typischerweise viel härter und starrer ist als das ihn umgebende Gefäßgewebe. Es könnten somit Schäden am Gefäßgewebe auftreten oder ungewollte biologische Reaktion des Gewebes hervorgerufen werden.

Damit die Aufweitung der Gefäße durch Stentimplantationen, sogenanntes Stenting, erfolgreich ist, sollte ein Stent so gewählt sein, dass es im Bereich des eingesetzten Stents nicht erneut zu Gefäßverengungen kommt.

Bei biodegradierbaren metallischen Stents ist in Tierversuchen insbesondere innerhalb der ersten zwei Wochen nach Implantation ein signifikanter Durchmesserverlust des Stents beobachtet worden. Der Erfolg des Stenting hängt somit auch davon ab, dass ein biodegradierbarer metallischer Stent nicht nach Implantation einen signifikanten Durchmesserverlust aufweist.

Die Aufgabe der vorliegenden Erfindung besteht darin, einen signifikanten Durchmesserverlust eines biodegradierbaren metallischen Stents nach Implantation zu verhindern bzw. zu vermindern.

Diese Aufgabe wird erfindungsgemäß durch einen biodegradierbaren metallischen Stent gelöst, der mit einem oder mehreren gefäßerweiternden Wirkstoffen ausgewählt aus der Gruppe der Calciumkanalblocker, der Nitrate, der Rho-Kinase-Inhibitoren, der Endothelinrezeptor-Antagonisten, der Serotonin-Antagonisten, der Adrenozeptor-Antagonisten, der Kaliumkanalöffner, der Angiotensin-Conversion-Enzym (ACE)-Inhibitoren, der muskulotropen und/oder neurotrop-muskulotropen Spasmolytika, der Vasodilatoren mit unbekanntem Wirkmechanismus sowie der oder den Wirkstoffen, die spasmogen wirksame Botenstoffe herunterregulieren in einer geeigneten Matrix so beschichtet ist, dass zeitnah zur Biodegradation des Stents nach dessen Implantation der oder die gefäßerweiternden Wirkstoffe gefäßerweiternd im Bereich der Stentimplantation wirken.

Im Sinne dieser Erfindung bedeutet zeitnah, dass der oder die gefäßerweiternden Wirkstoffe in einer Zeitspanne von kurze Zeit vor bis kurze Zeit nach dem Beginn der Degradation des implantierten Stents im Bereich der Stentimplantation gefäßerweiternd wirken und solange die gefäßerweiternde Wirkung fortsetzen, bis die Biodegradation des Stents abgeschlossen ist, vorzugsweise bis die durch die Biodegradation des Stents freigesetzte Konzentration an Hydroxidionen nicht mehr ausreicht einen Gefäßspasmus auszulösen bzw. zu vermitteln, weiter bevorzugt innerhalb von 4 Monaten nach Stentimplantation, mehr bevorzugt innerhalb von 1 bis 8 Wochen, besonders bevorzugt innerhalb von 1 bis 3 Wochen nach Stentimplantation.

Die Erfindung beruht auf der von den Erfindern gefundenen, neuen Erkenntnis, dass der Grund für den signifikanten Durchmesserverlust von biodegradierbaren metallischen Stents nach Implantation ein Gefäßspasmus, insbesondere ein dauerhafter Gefäßspasmus im Bereich des implantierten Stents darstellt. Ein solcher Gefäßspasmus beginnt nach neuesten Erkenntnissen zwischen 1 und 3 Wochen nach der Implantation. Dagegen wurde nach einer Implantation von biodegradierbaren polymeren Stents oder dauerhaften metallischen Stents kein solches Phänomen beobachtet.

Aufbauend auf dieser neuen Erkenntnis haben die Erfinder weiter gefunden, dass eine mögliche Ursache für den Gefäßspasmus die freigesetzten Metallionen und/oder die sich bildenden Hydroxidionen sind, die im Bereich des implantierten biodegradierbaren metallischen Stents bei der Biodegradation des metallischen Stents auftreten.

Eine Erklärungsmöglichkeit hierfür besteht darin, dass nach Implantation durch die Biodegradation der metallischen Stents Metall- und Hydroxidionen in einer solchen Konzentration im Gefäßbereich des Stents freigesetzt vorliegen, dass diese Konzentration vor allem an Hydroxidionen ausreicht, um die Calciumionenkanäle des Gefäßgewebes, wahrscheinlich die Calciumionenkanäle des L-Typs, im Bereich des Stents zu öffnen und so zu einem Einstrom von Calciumionen aus dem extrazellulären Raum in die glatte Gefäßmuskelzelle zu führen. Es wird vermutet, dass durch den Anstieg der intrazellulären Calciumionen-Konzentration in der Gefäßmuskelzelle die eigentlichen Reaktionen ausgelöst werden, welche einen solchen Gefäßspasmus vermitteln.
Nach neuesten Erkenntnissen hat sich gezeigt, dass neben soeben erwähnten Calciumkanälen auch die sogenannten TRP-Kanäle (Transient Receptor Potential) eine nicht ganz unwesentliche Rolle bei der Proliferation, Intima-Verdickung, Angiogenese und der Bildung eines Gefäßspasmus spielen. Vor allem im Blick sind die TRP-C1-, TRP-C3-, TRP-C4- und TRP-C6-Kanäle, da diese in besonderem Maße von Hydroxid- und einigen Metallionen beeinflusst werden.

Diese Erklärungstheorie wird durch Erkenntnisse aus anderen technischen Gebieten gestützt, nämlich dass der pH-Wert einen Einfluss auf die Kontraktilität von Blutgefäßen haben kann. Diese Erkenntnis wurde in den 80er Jahren verwendet, um bei symptomatischen Patienten derartige Spasmen auszulösen (Weber S: "Systemic alkalosis as a provocative test for coronary artery spasm in patients with infrequent resting chest pain"; Am Heart J. 1988, 115:54-9). Die zu Grunde liegenden Regelungsprozesse werden in Lehrbüchern der Pharmakologie beschrieben (z.B. Mutschler *et al*.; "Arzneimittelwirkungen: Lehrbuch der Pharmakologie und Toxikologie"). Die allgemeine Erkenntnis geht dahin, dass Hydroxidionen einen Einfluss auf die Calciumionenkanäle des L-Typs von glatten Gefäßmuskelzellen haben und diese öffnen können, wodurch es zu einem Einstrom von Calciumionen aus dem extrazellulären Raum in die glatte Gefäßmuskelzelle kommt. Es wird ferner beschrieben, dass in einer ruhenden Gefäßmuskelzelle die Konzentration an freien Calciumionen im Vergleich zum Extrazellularraum nur ca. 1:10.000 beträgt und durch den äußeren Reiz der Hydroxidionen die intrazelluläre Calciumionen-Konzentration kurzfristig auf ca. 1:1.000 ansteigt. Intrazellulär werden die Calciumionen an Calciumbindende Proteine (unter anderem Calmodulin) gebunden und durch in dieser Weise aktivierte Proteine in der Gefäßmuskelzelle die eigentlichen Reaktionen, nämlich die Vermittlung eines Gefäßspasmus, ausgelöst werden.

Groschner et al. ("Intracellular pH as a Determinant of Vascular Smooth Muscle Function", J Vasc Res 2006; 43:238-250) befasst sich mit der intrazellulären pH-Wert Erhöhung und deren direkten Auswirkung auf die Kontraktiliät, Wachstum und Proliferation von glatten Gefäßmuskelzellen. In Bezug auf die Kontraktilität wird festgestellt, dass keine allgemeingültige Aussage für eine intrazelluläre pH-Wert Regulation gemacht werden kann, sondern dass eher ein komplexes Beziehungsgeflecht zwischen intrazellulärem pH-Wert und Gefäßtonus besteht. Es wird ferner beschrieben, dass die Auswirkungen einer intrazellulären pH-Wert Erhöhung für unterschiedliche Gefäße stark von der Art der Gefäße, aber auch von Versuchsdurchführungen abzuhängen scheinen. In großen Arterien scheint der Ruhetonus fest an den pH-Wert gekoppelt zu sein, wobei eine Alkalose eine Vasokonstriktion auslöst. Im Unterschied dazu scheinen kleine Arterien und Arteriolen unempfindlicher gegenüber pH-Wert-Schwankungen zu sein.

*In vitro* Tests haben zudem gezeigt, dass derartige Gefäßspasmen eine Kraft von bis zu 2,5 bar entwickeln können.

Der vorliegende Stand der Technik offenbart allerdings nicht, dass zum einen biodegradierbare metallische Stents nach Implantation im Bereich der Stentimplantation einen signifikanten Durchmesserverlust aufweisen, dass diese signifikante Durchmesserverkleinerung durch einen Gefäßspasmus vermittelt wird und dass wahrscheinlich dieser Gefäßspasmus durch freigesetzte Metallhydroxidionen aus dem biodegradierbaren Stent (teilweise) ausgelöst und/oder vermittelt wird.

Somit nimmt der Stand der Technik nicht die oben dargestellte Erkenntnis der Erfinder über die Ursache der beobachteten Gefäßverengungen vorweg, nämlich den Gefäßspasmus sowie dessen Ursache, nämlich die erhöhte Konzentration von Metallhydroxidionen als Folge der Stentdegradation, sondern stellt einen Erklärungsansatz dar, der sich im Nachhinein in Anbetracht der Erkenntnisse der Erfinder anbietet.

Entsprechend hat sich überraschender Weise vorteilhaft gezeigt, dass durch die Freisetzung der Metallhydroxidionen durch Degradation des metallischen Stents sowie die zeitnahe gefäßerweiternde Wirkung im Bereich der Stentimplantation eines oder mehrerer gefäßerweiternden Wirkstoffen die über die freigesetzten Metallhydroxidionen (teilweise) ausgelösten und/oder vermittelten Reaktionen verhindert bzw. vermindert werden können.

Folglich kann die gefäßerweiternde Wirkung des oder der Wirkstoffe zeitnah zur Bildung und Freisetzung der Metallhydroxidionen aus einem erfindungsgemäßen biodegradierbaren metallischen Stent einen signifikanten Durchmesserverlust des biodegradierbaren metallischen Stents verhindern bzw. vermindern.

Ein erfindungsgemäßer biodegradierbarer metallischer Stent ist vorzugsweise zylindrisch und expandierbar.

Gefäße, die für solche Stentimplantationen geeignet sind, sind menschliche oder tierische Blutgefäße, insbesondere Arterien und Venen, hiervon sind insbesondere Arterien bevorzugt. Bevorzugt sind insbesondere Stentimplantationen in großen Arterien.

Gefäßlumen im Sinne dieser Erfindung bedeutet, dass der Hohlraum eines Blutgefäßes gemeint ist.

Im Sinne dieser Erfindung bedeutet Elution oder Eluierung des oder der Wirkstoffe, dass der oder die Wirkstoffe aus der Trägermatrix freigesetzt werden.

Der Regelkreis pH-Wert/Ionenkanäle/Calcium-Haushalt/Gefäßkontraktion kann an verschiedenen Stellen durch geeignete gefäßerweiternde Wirkstoffe unterbrochen werden.

Geeignete gefäßerweiternde Wirkstoffe, die direkt vom beschichteten Stent an das Gefäßlumen, bevorzugt das den Stent umgebende Gefäßlumen und angrenzende Gebiete, weiter bevorzugt die an den Stent angrenzende Gefäßwand, freigesetzt werden, werden aus der Gruppe der Calciumkanalblocker, der Nitrovasodilatatoren, der Rho-Kinase-Inhibitoren, der Endothelinrezeptor-Antagonisten, der Serotonin-Antagonisten, der Adrenozeptor-Antagonisten, der Kaliumkanalöffner, der Angiotensin-Conversion-Enzym (ACE)-Inhibitoren, der muskulotropen und/oder neurotrop-muskulotropen Spasmolytika, der Vasodilatoren mit unbekanntem Wirkmechanismus (z.B. Hydrazinderivate, Cicletanin) des Wirkstoffes oder der Wirkstoffe, die spasmogen wirksame Botenstoffe, wie z.B. Endothelin, Thromboxan, Serotonin, 5-HT, ADP (Vasopressin) und PAF (Platelet activating factor), herunterregeln, insbesondere gentherapeutisch heruntergeregeln, der Phosphodiesterase-5-Hemmer (PDE-5) wie z.B. Sildenafil (Viagra), Tadalafil (Cialis) oder Vardenafil (Levitra, Vivanzia), TRP-Hemmer oder -aktivatoren, sowie des Wirkstoffes oder der Wirkstoffe; die TRP-Kanäle aktivieren oder inhibieren können, ausgewählt.

### ● Calciumkanalblocker

Calciumkanalblocker verhindern den direkten Einstrom von Calciumionen in die Gefäßmuskelzelle. In zahlreichen *in vitro* Versuchen konnte gezeigt werden, dass pHinduzierte Spasmen auf diese Weise weitgehend vermieden werden können. Bevorzugt sind Calciumkanalblocker mit Wirkung auf die L-Kanäle.

Geeignete Calciumkanalblocker sind beispielsweise
● Dihydropyridine (Substanzen vom Nifedipin-Typ):
   Nifedipin, Nisoldipin, Nicardipin, Nitrendipin, Nimodipin, Felodipin, Isradipin, Nilvadipin, Amlodipin, Lercanidipin, Lacidipin, etc.
● Phenylalkylamine (Substanzen vom Verampamil Typ):
   Verampamil, Gallopamil, etc.
● Benzothiazepine (Substanzen vom Diltiazem-Typ):
   Diltiazem, etc.
● Sonstige:
   Fendilin

Bevorzugt sind Lercanidipin, Lacidipin, Amlodipin und Nitrendipin.

### ● Nitrovasodilatoren

Organische Nitrate, Nitrite und Aminosäuren, die einer metabolischen Umwandlung zu NO (der eigentlichen Wirksubstanz) unterliegen, werden als Nitrovasodilatoren bezeichnet. Somit sind Nitrate typischerweise Prodrugs. NO stimuliert die cytosolischer Guanylatcyclase, welche die Bildung von cyclischem Guanosinmonophosphat (cGMP) aus Guanosintriphosphat (GTP) katalysiert. CGMP seinerseits bewirkt dann die Abnahme der intrazellulären Calciumionen-Konzentration und dadurch eine Erniedrigung des Gefäßtonus.

Geeignete Wirkstoffe sind beispielsweise:
Glyceroltrinitrat (Nitroglycerin), Isosorbiddinitrat, Isosorbid-5-mononitrat, Pentaerythrityltetranitrat, Molsidomin, Linsidomin, Nicorandil, Nitroprussid-Natrium, Aminosäure L-Arginin und Polypeptide, die ganz oder teilweise aus L-Arginin bestehen, etc.

### ● Rho-Kinase-Inhibitoren

Die Rho-Kinase gehört zur Familie der Serin/Threonin Kinasen und wird durch verschiedene vasoaktive Mediatoren, wie Katecholamine, UII, Thromboxan und Serotonin aktiviert. Sie spielt eine Schlüsselrolle in der Gefäßkontraktion des glatten Muskels. Die Rho-Kinase induzierte Kontraktion ist in allen Gefäßbetten der verschiedenen untersuchten Tierspezies (Ratte, Maus, Kaninchen, Schwein) induzierbar und durch selektive Rho-Kinase Inhibitoren konzentrationsabhängig hemmbar (Steioff, Kerstin, "Multiple Wirkungen der Rho-kinas: Neue Möglichkeiten zur Therapie von Bluthochdruck), Doktorarbeit 2005, Frankfurt am Main, http://publikationen.ub.uni-frankfurt.de/volltexte/2005/1579/pdf/SteioffKerstin.pdf).

Geeignete Rho-Kinase Inhibitoren sind beispielsweise:
Fasudil (HA 1077), Fasudil-Derivat (HA 1152) und Y 27632 etc. (insbesondere beschrieben in Hu et al, "Rho-kinase inhibitors as potential therapeutic agents for cardiovascular diseases", Cur. Opin. Investig. Drugs 2003; 4 (9):1065-1075).

### ● muskulotrope oder neurotrop-muskulotrope Spasmolytika

Unabhängig von der vegetativen Innervation kann die glatte Muskulatur durch direkte Einwirkung von geeigneten Wirkstoffen auf die glatten Muskelzellen erschlafft werden. Auf diese Weise wirkende Wirkstoffe werden als muskulotrope oder papaverinartige Spasmolytika bezeichnet.

Geeignete muskulotrope Spasmolytika sind beispielsweise:
Papaverin, Tiropramid etc.

Eine Zwischenstellung zwischen den Parasympatholythika und den muskulotropen Spasmolytika nehmen Wirkstoffe ein, die sowohl neurotrope (parasympatholytische) als auch muskulotrope spasmolytische Eigenschaften besitzen.

Geeignete neurotrop-muskulotrope Spasmolytika sind beispielsweise: Drofenin, Mebeverin, Oxybutynin, Propiverin etc.

### ● Endothelinrezeptor-Antagonisten

Endotheline (ET-1, ET-2 und ET-3) stimulieren den ET_{A}-Rezeptor auf der glatten Gefäßmuskulatur und führen zu einer Gefäßkonstriktion. Dabei ist die vasokonstriktorische Eigenschaft von ET-1 etwa 10-mal größer als die von Angiotensin II. Endothelzellen reagieren auf eine Schädigung (z.B. durch hohe pH-Werte) durch Ausschüttung von Endothelinen. Endothelinrezeptor-Antagonisten blockieren die ET_{A}- und die ET_{B}-Rezeptoren und verhindern so eine übermäßige Gefäßkontraktion. Ein hierfür geeigneter Endothelinrezeptor-Antagonist ist z.B. Bosentan.
Es hat sich nun nach neuesten Erkenntnissen herausgestellt, dass auch eine selektive Blockung der ET_{A}-Rezeptoren Vorteile bietet.

### ● Kaliumkanalöffner:

Die Öffnungswahrscheinlichkeit von Kaliumkanälen in der Zellmembran (z.B. in der glatten Gefäßmuskulatur arterieller Blutgefäße) bestimmt die Höhe des Ruhepotentials. Mit steigender Öffnungswahrscheinlichkeit wird das Ruhemembranpotential in Richtung des Kalium-Gleichgewichtpotentials verschoben, die Membran hyperpolarisiert. Als Folge sinkt der Calciumeinstrom durch spannungsabhängige Calciumkanäle., was zu einer Abnahme des intrazellulären Calciums und damit zur Vasodilatation führt
Geeignete Kaliumkanalöffner sind z.B. Diazoxid, Monoxidil, Nicorandil, Pinacidil, Levcromakalim.

### ● Vasodilatoren mit unbekannten Wirkmechanismus

Der Wirkmechanismus der Hydrazinderivate ist bis heute nicht bekannt. Die wesentliche hämodynamische Wirkung besteht in einer Abnahme des peripheren Gefäßwiderstands. Der Wirkmechanismus von Cicletanin ist ebenfalls unbekannt. Es wirkt unter anderem vasodilatorisch, wahrscheinlich durch Stimulation der Muscarinrezeptoren auf endothelzellen und durch Hemmung spannungsabhängiger Calciumkanäle in der Gefäßmukulatur.
Geeignete Substanzen sind z.B. Hydralazin, Dihydralazin und Cicletanin

### ● Serotonin-Antagonisten

Substanzen, die die Wirkung des Spasmogens Serotin antagonisieren werden als Serotonin-Antagonisten bezeichnet. Häufig haben diese Substanzen ebenfalls einen einfluss auf die Calciumkanäle in intrazellulären Calciumspeicher.
Geeignete Substanzen sind z.B. Cinnarizin und Flunarizin.

### ● Angiotensin-Conversion-Enzym (ACE)-Inhibitoren

Geeignete Wirkstoffe sind z.B.:
Benazepril, Captopril, Cilazapril, Enalapril, Fosinopril, Imidapril, Lisinopril, Moexipril, Perindopril, Quinapril, Ramipril, Spirapril, Trandolapril, etc.

### ● Adrenozeptor-Antagonisten

Geeignete Wirkstoffe sind z.B.:
Doxazosin, Prazosin, Uradipil, Sotalol, Propanolol, Pindolol, Carvedilol, Metoprolol, Atenolol, Talinolol, Bisoprolol, Nebivolol, Betaxolol etc.

### ● PDE-5-Hemmer

Geeignete Wirkstoffe sind z.B.:
Sildenafil (Viagra), Tadalafil (Cialis), Vardenafil (Levitra, Vivanzia)

Geeignete Wirkstoffe, die direkt vom beschichteten Stent an das Gefäßlumen, bevorzugt das den Stent umgebende Gefäßlumen und angrenzende Gebiete, weiter bevorzugt an die den Stent umgebende und angrenzende Gefäßwand abgegeben werden, weisen in bevorzugten Ausgestaltungen folgende Eigenschaften auf:

### ● pH-Wert Stabilität

Da die Erhöhung des pH-Wertes, die gerade die lokalen Gefäßspasmen auslöst, ebenfalls zu chemischen Veränderungen, insbesondere zur Deprotonierung des geeigneten Wirkstoffes führen kann, liegt ein geeigneter Wirkstoff bevorzugt bei pH kleiner gleich 11, mehr bevorzugt bei pH kleiner gleich 12 in seiner pharmazeutisch aktiven Form vor, bzw. erfolgt die chemische Umwandlung in diesem pH-Bereich hinreichend langsam.

### ● Lipophilie

Ein bevorzugter Wirkstoff ist ebenfalls lipophil, da lipophile Wirkstoffe die Gefäßwand als Speicher nutzen können und daher länger in den lipophilen Gefäßkompartimenten, die den Stent umgeben sowie in angrenzenden Gebieten hiervon, verbleiben kann und so zu einer dauerhaften Dilatation führen kann, auch wenn die primäre Wirkstoffabgabe durch den Stent bereits weitgehend abgeschlossen ist (Depotwirkung).

So zeigt ein Sirolimus eluierender Stent, der seinen Wirkstoff innerhalb von 7 Tagen abgibt im Humanversuch über 6 und 24 Monate die gleiche Wirksamkeit wie ein Stent, bei dem Sirolimus über einen Zeitraum von 30 Tagen abgegeben wird (Sousa at al., "Lack of neointimal proliferation after implantation of sirolismus-coated stents in human coronary arteries: a quantitative coronary angiography and three-dimensional intracascular ultrasound study", Circulation, 2001; 103:192-195; Sousa et al., "Twoyear angiographic and intravascular ultasound follow-up after impantation of sirolismus-eluting stent in human coronary arteries", Circulation, 2003, 107: 381-383).

Bevorzugt sind insbesondere lipophile Calciumkanalblocker ausgewählt aus der folgenden Gruppe: Lercanidipin, Lacidipin, Amlodipin und Nitrendipin.

### ● Keine Toleranzentwicklung

Bei der Verwendung eines geeigneten Wirkstoffes sollte es bevorzugt nicht zu einer Toleranzentwicklung kommen.

Als Beispiel für einen Wirkstoff, der eine solche Toleranz, eine sogenannte Nitrattoleranz, aufweist, dient Glycerintrinitrat. Nach ca. 24 Stunden lässt die Wirksamkeit von Glycerinnitrat bereit nach, wobei die Prozesse, die hierfür verantwortlich sind noch nicht vollständig geklärt sind (Munzel et al., "Explaining the phenomenon of nitrate tolerance", Circ Res., 2005 Sep 30, 97(7): 618-628).

Bevorzugt sind insbesondere Nitrate ausgewählt Gruppe: Pentaerythryltetranitrat, Molsidomin und Linsidomin, da diese Substanzen NO nichtenzymatisch freisetzen, was sehr wahrscheinlich die Ursache für die nicht vorhandene Toleranzentwicklung darstellt.

Ein weiterer Wirkstoff, der ebenfalls keine Toleranzentwicklung aufweist, ist L-Arginin. Obwohl dieser Stoff kein Nitrat ist, setzt er auch NO frei.

### ● Hohe Wirksamkeit

Bevorzugt sind Wirkstoffe, die eine hohe Wirksamkeit aufweisen, damit für die Beschichtung des Stents wenig Wirkstoff benötigt wird, um eine möglichst lange Wirkdosis aufrecht zu erhalten.
Hierdurch kann ein derart bevorzugter Wirkstoff dazu führen, dass die Schichtdicke der beschichteten Stents im Vergleich zur Schichtdicke von weniger wirksamen Wirkstoffen nicht so dick ist.

### ● Lange Halbwertszeit

Bevorzugte Wirkstoffe weisen zudem eine lange Halbwertszeit auf.

Ein in dieser Weise bevorzugter Wirkstoff führen dazu, dass die Schichtdicke des beschichteten Stents im Vergleich zur Schichtdicke Wirkstoffen mit geringerer Halbwertszeit nicht so dick ist.

Die bevorzugten Ausführungsformen werden zudem in den Unteransprüchen beschrieben.

In einer bevorzugten Ausführungsform entspricht die Elutionszeit des oder der gefäßerweiternden Wirkstoffe aus der Matrix [T_{E}(W)] der Degradationszeit des erfindungsgemäßen Stents [T_{D}(S)].

Diese Ausgestaltung eines solchen Degradations- bzw. Elutionsverhaltens kann dadurch realisiert werden, dass der erfindungsgemäße Stent teilweise mit der Matrix beschichtet ist. Ein Beispiel für einen in dieser Weise beschichteten Stent wird in WO 2005/102222 offenbart.

Bevorzugt eluieren der oder die gefäßerweiternden Wirkstoffe über einen Zeitraum von 4 Monaten, mehr bevorzugt über einen Zeitraum von mindestens 3 bis 8 Wochen beginnend mit der Stentimplantation.

In einer weiteren bevorzugten Ausführungsform entspricht die Elutionszeit des oder der Wirkstoffe aus der Matrix [T_{E}(W)] der Degradationszeit des Wirkstoffdepots der Matrix auf erfindungsgemäßen Stent [T_{D}(D)] und
[T_{D}(D)] und [T_{E}(W)] sind jeweils kleiner als die Degradationszeit des Stents [T_{D}(S)]
und
[T_{D}(S)] ist kleiner oder gleich der Elutionszeit des oder der Wirkstoffe aus der Gefäßwand [T_{E}(G)].

Diese Ausgestaltung eines solchen Degradations- bzw. Elutionsverhaltens kann durch die klassische Stentbeschichtung realisiert werden. Hierbei kommt es zu einer vollständigen Beschichtung des erfindungsgemäßen Stents sowohl auf der Innenseite, die dem Gefäßlumen zugewandt ist, als auch auf der muralen Seite, der Außenseite, die der Gefäßwand zugewandt ist. Die Matrix stellt mithin ein Wirkstoffdepot dar. Diese Ausgestaltung ist bevorzugt geeignet für ein oder mehrere lipophile gefäßerweiternde Wirkstoffe, die aus der Matrix eluieren und sich in der Weise in den Zellen der Gefäßwand, welche den Stent umgibt, anreichern, so dass dort ein Wirkstoffdepot entsteht. Der oder die gefäßerweiternden Wirkstoffe eluieren dann aus dem Wirkstoffdepot der Gefäßwand zeitnah zur Biodegradation des erfindungsgemäßen Stents und damit zur Freisetzung von Hydroxidionen aus dem erfindungsgemäßen Stents.

In einer weiteren bevorzugten Ausführungsform ist die Degradationszeit des Stents [T_{D}(S)] kleiner als die Elutionszeit des oder der Wirkstoffe aus der Gefäßwand [T_{E}(G)] oder kleiner als die Elutionszeit des oder der Wirkstoffe aus der Matrix des Stents [T_{E}(W)], wobei die [T_{E}(G)] der [T_{E}(W)] entspricht.

Diese Ausgestaltung eines solchen Degradations- bzw. Elutionsverhaltens kann durch eine "ablösbare" Beschichtung der Matrix von dem erfindungsgemäßen Stents realisiert werden.

Eine in dieser Weise "ablösbare" Beschichtung eines erfindungsgemäßen Stents zeichnet sich insbesondere dadurch aus, dass die Matrix auf einem hierzu vorgesehenen Beschichtungsbereich der Oberfläche des Grundkörpers derart aufgebracht ist, dass
● der Beschichtungsbereich in einen unbeschichteten Teilbereich und einen mit der Matrix beschichten Teilbereich gegliedert ist, wobei der beschichtete Teilbereich 5 bis 80 % der Oberfläche des erfindungsgemäßen Stentes bedeckt;
● ein Abstand von einem beliebigen Punkt der Oberfläche im beschichteten Teilbereich zum nächstgelegenen unbeschichteten Teilbereich kleiner ist als 60 µm; und
● ein Abstand von einem beliebigen ersten Randpunkt der Oberfläche im beschichteten Teilbereich zu dem einen zweiten Randpunkt im selben beschichteten Teilbereich, der von dem ersten Randpunkt am weitesten entfernt ist, höchstens 400 µm beträgt.

Durch eine solche Beschichtung ist es möglich, die Degradationsverhalten der Matrix und des Stents zu Entflechten und somit die Elution der Wirkstoffe sowie die Vorgänge bei der Degradation genauer abzustimmen und ggf. notwendige Modifikationen nur auf einen Teil des Systems zu beschränken. Aufgrund der Grundgerüstdegradation werden sich die beschichteten Teilbereiche von der Oberfläche des Grundkörpers lösen und, falls im Gewebskontakt befindlich, in das umgebende Gewebe einwachsen. Dort fungieren sie als lokale Wirkstoffdepots, die weder lokal noch bezüglich der Elutions- und Degradationsprozesse im Kontakt mit dem Grundgerüst des Implantats stehen.

In den vorstehenden bevorzugten Ausführungsformen können, sofern zwei, drei oder mehrere gefäßerweiternde Wirkstoffe in der Matrix inkorporiert sind, diese unabhängig voneinander eluieren.

In einer weiteren bevorzugten Ausführungsform umfasst die Matrix eines erfindungsgemäßen Stents zusätzlich ein oder mehrere Wirkstoffe aus der Gruppe der antiinflammatorischen und/oder antiproliferativen Wirkstoffe und/oder Antisensenukleotide und/oder Bisphosphonate und/oder Antikörper und/oder Progenitorzellen.

Eine geeignete Matrix im Sinne dieser Erfindung kann somit den oder die Wirkstoffe enthalten oder umgeben, beispielsweise in Schäumen, in Kanalsystemen und/oder in Schichtsystemen und/oder insbesondere die Freisetzung des oder der Wirkstoffe steuern. Eine Matrix im Sinne dieser Erfindung kann zudem die Struktur des Stents unterstützen, strukturelle Intaktheit und/oder strukturelle Barrieren bieten. Die Freisetzung der Wirkstoffe aus einer geeigneten Matrix kann über das Matrixmaterial oder die Art der Beschichtung gesteuert werden, wie sie im Stand der Technik bereits beschrieben ist.

Eine geeignete Matrix im Sinne der vorliegenden Erfindung kann ausgewählt werden aus der Gruppe der
● Polymere, wie beispielsweise Polylactide (PLA poly lactide acid), Methylmethacrylat (MMA), Polyhydroxybuttersäure (PHB poly hydroxy butyrat), Poly(orthoester) (POE), Polypeptide, Polysaccharide, Phosphorylcholin (PC), Hyaluronsäure (HA hyaluronic acid), Cholesterin oder
● Fette, wie beispielsweise natürliches oder modifiziertes Sojaöl etc..

Bevorzugte Matrixmaterialien sind bioresorbierbar. Bevorzugt werden die geeigneten Matrixmaterialien nicht vor der vollständigen Degradation des metallischen Stents abgebaut.

Die Menge der Matrixbeschichtung ist abhängig von der Wirksamkeit der gefäßerweiternden Wirkstoffe. So liegt die Wirkstoffmenge auf einem Stent mit 10mm Länge und einem Durchmesser im dilatierten Zustand von ca. 3mm bevorzugt im Bereich 1 bis 500 µg, mehr bevorzugt im Bereich 10 bis 200 µg. Generell kann ein Stent je nach Anwendungsbereich eine Länge von 8 bis 80 mm und einen Durchmesser von 2 bis 12 mm aufweisen. Die Wirkstoffmenge wird je nach Länge und Durchmesser angepasst.

Unter Beschichtung im Sinne der vorliegenden Erfindung ist eine Ummantelung des biodegradierbaren metallischen Stents und/oder eine Befüllung und/oder eine Beladung von Aussparungen, vorzugsweise Löchern bzw. Kavitäten und/oder Körbchen im und/oder am Stent mit einer geeigneten Matrix, die einen oder mehrere gefäßerweiternde Wirkstoffe und gegebenenfalls einen oder mehrere weitere Wirkstoffe enthält, in der Weise zu verstehen, wobei der biodegradierbare metallische Stent in der Weise beschichtet wird, dass der metallische Stent nach Implantation in ein Gefäßlumen biodegradieren und Metallhydroxidionen freisetzen kann und zeitnah der oder die Wirkstoffe gefäßerweiternd.

Die Erfindung soll nun anhand von Ausführungsformen mit Bezug auf die Figuren näher erläutert werden. Der Umfang der Erfindung wird jedoch nicht hierauf beschränkt.

Üblicherweise weisen Stents einen Grundkörper aus Metall auf, der von einer Vielzahl von Stegen gebildet ist.

Die Figuren zeigen beispielhaft Abschnitte von Stegen eines im Übrigen beliebigen Grundkörpers eines Stents. Von den Figuren ist:
Fig. 1a) ein Steg mit Löchern 11 eines Stentgrundkörpers.
Fig. 1b) ein Steg mit Kavitäten 15 eines Stentgrundkörpers.
Fig. 1c) ein Steg mit Körbchen 17 eines Stentgrundkörpers.
Fig. 2) ein Steg mit einer "ablösbaren" Beschichtung 18 eines Stentgrundkörpers.

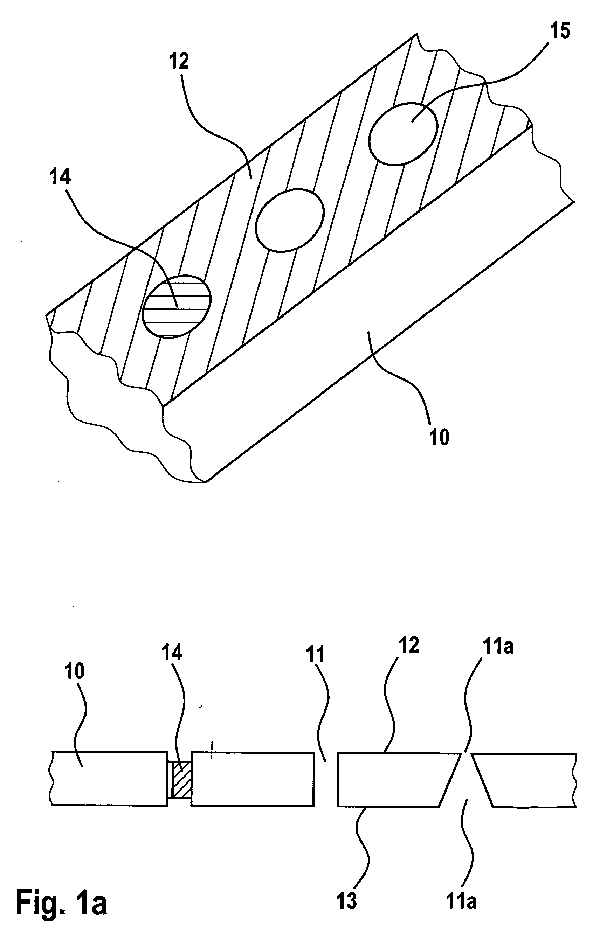

In einer bevorzugten Ausführungsform ist die geeignete erfindungsgemäße Beschichtung mit geeigneten Wirkstoffen ganz oder teilweise in Löchern bzw. Kavitäten eines biodegradierbaren Stents gefüllt. Solche Löcher bzw. Kavitäten werden in Figuren 1a), 1b) und 1c) dargestellt.

Figuren 1a), 1b) und 1c) zeigen einen Abschnitt aus einem Grundkörper 10 eines Stents, der aus einem biodegradierbaren Material geformt ist. Der metallische Werkstoff bildet ein filigranes Gerüst aus miteinander verbundenen Stegen.

Figur 1a) zeigt einen Steg 10, der in einer solchen Weise durchbohrt ist, dass sich im wesentlichen zylindrische Löcher 11 in dem Steg ergeben, die von einer Außenoberfläche 12 zur einer Innenoberfläche 13 führen. Diese Löcher 11 können mit einer erfindungsgemäßen wirkstoffenthaltenden Polymerbeschichtung 14 ganz oder teilweise befüllt sein.

Ein in dieser Weise geeigneter Stent wird bereits in WO 2005/102222 (Conor Medsystems, Inc.) als sogenannter "Conor-Stent" beschrieben und kann als Grundkörper für den erfindungsgemäßen Stent und die erfindungsgemäße Beschichtung dienen.

Alternativ oder kumulativ können zu den Löchern 11 können ebenfalls konisch geformte Löcher 11a) vorliegen, wobei sich diese zu der Außenoberfläche 12, welche die murale Außenseite des Stents bildet, verjüngen, damit beim Dillatieren des Stents die Polymerbeschichtung 14 in dem Loch 11a) gehalten wird.

Demnach können ausschließlich zylindrisch geformte Löcher 11 oder konisch geformte Löcher 11a) oder eine beliebige Kombination von zylindrisch geformten Löchern 11 und konisch geformten Löchern 11a) in einem erfindungsgemäßen Stent vorliegen.

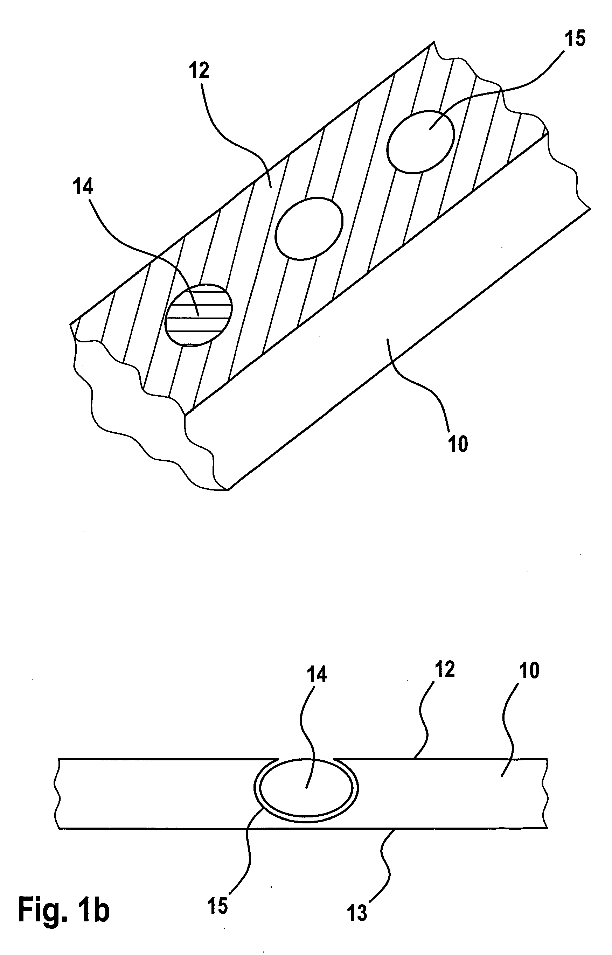

Figur 1b) zeigt einen Steg 10, der in einer solchen Weise bearbeitet ist, dass sich Kavitäten 15 in dem Steg 10 ergeben, die in Richtung der Außenoberfläche 12 des Steges eine Öffnung aufweisen und in Richtung der Innenoberfläche 13 geschlossen sind. Diese Kavitäten 15 können mit einer erfindungsgemäßen wirkstoffenthaltenden Polymerbeschichtung 14 ganz oder teilweise befüllt sein.

Demnach können ausschließlich Kavitäten 15, aber auch beliebige Kombinationen von Kavitäten 15 und zylindrisch geformten Löchern 11 und/oder konisch geformten Löchern 11a) in einem erfindungsgemäßen Stent vorliegen.

Kavitäten können auch in einer porösen Oberfläche hergestellt werden. Solche Porösitäten werden durch die Behandlung der Oberfläche oder durch Konversionsschichten hergestellt. Ein solches Verfahren zur Herstellung einer Konversionsschicht und ein nach dem Verfahren hergestelltes Implantat ist Patentanmeldung DE 10 2006 060 501.2 der Anmelderin aufgezeigt. Ein weiteres Verfahren zur porösen Beschichtung eines magnesiumhaltigen Gegenstandes mit einer Konversionsschicht ist in der WO 00/56950 A1 dargestellt. Der Inhalt beider Schriften ist hier in vollem Umfang beinhaltet.

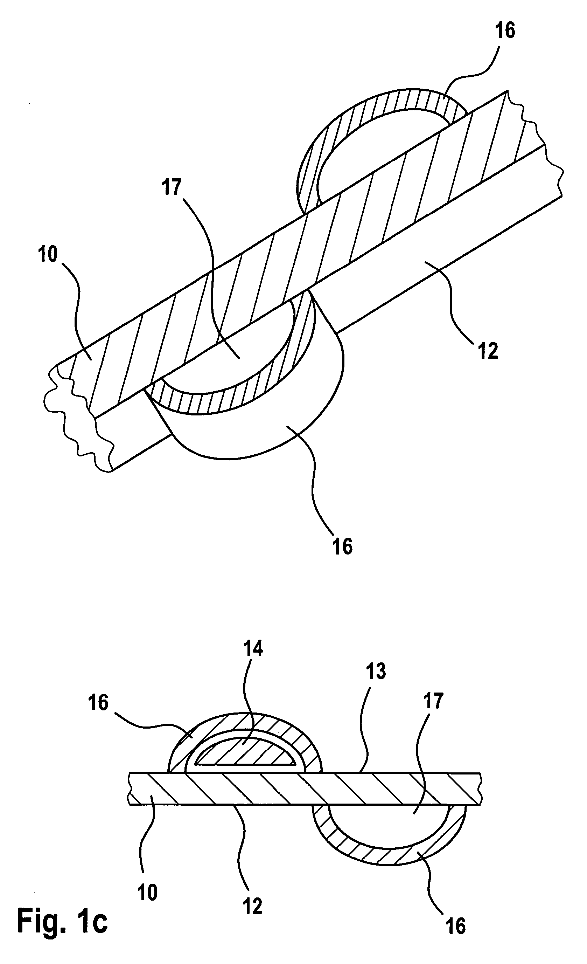

Figur 1c) zeigt einen Steg 10, der in einer solchen Weise bearbeitet ist, dass Körbchen 16 an den Steg 10 auf der Außenoberfläche 12 und/oder der Innenoberfläche 13, bevorzugt an der Außenseite 12 angegliedert sind. Durch diese Körbchen 16 wird eine Öffnung 17 gebildet, die mit der erfindungsgemäßen wirkstoffenthaltenden Polymerbeschichtung 14 ganz oder teilweise befüllt sein können.

Demnach können ausschließlich Körbchen 16, aber auch beliebige Kombinationen von Körbchen 16 und Kavitäten 15 und/oder zylindrisch geformten Löchern 11 und/oder konisch geformten Löchern 11a) in einem erfindungsgemäßen Stent vorliegen.

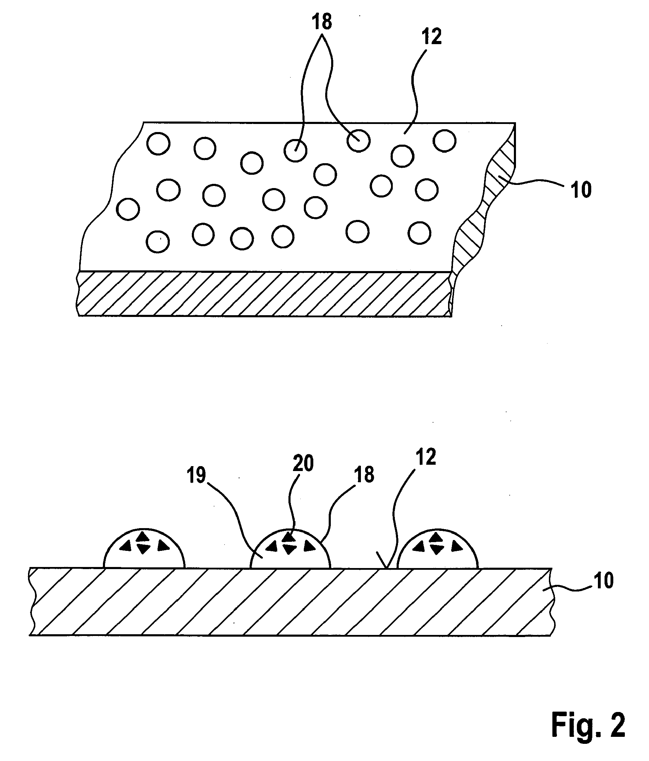

In einer weiteren bevorzugten Ausführungsform kann die geeignete erfindungsgemäße Beschichtung mit geeigneten Wirkstoffen als "ablösbare" Beschichtung beschichtet sein. Eine solche Ausführungsform wird durch Figur 2 dargestellt.

In Figur 2) wird auf eine äußere Außenoberfläche 12 des Stegs 10 eine wirkstoffenthaltene Polymerbeschichtung aufgebracht. Wie ersichtlich, ist der Beschichtungsbereich in einen unbeschichteten Teilbereich und einen beschichteten Teilbereich gegliedert.

Die wirkstoffenthaltene Polymerbeschichtung ist vorliegend als eine Vielzahl von Beschichtungsinseln 18 realisiert, die aus einer biodegradierbaren Trägermatrix 17 und zumindest einer in die Trägermatrix 19 eingebetteten Wirkstoff 20 (hier dargestellt als Dreieck) bestehen. Die Beschichtungsinseln 18 sind derart auf der Oberfläche 12 des Grundkörpers 10 aufgebracht, dass der beschichtete Teilbereich, also die Beschichtungsinseln 18, etwa 10 - 15 % der Außenoberfläche 12 des Beschichtungsbereichs bedecken.

Der Steg 10 besteht vorliegend aus der Magnesiumlegierung WE43 und die Trägermatrix ist hochmolekulares Poly-L-lactid (Molmasse > 500 kD). Eine Abbaugeschwindigkeit des polymeren Materials der Trägermatrix 15 beträgt etwa das 10 - 15fache im Vergleich zur Abbaugeschwindigkeit des Materials des Grundkörpers 10.

Die einzelnen Beschichtungsinseln weisen einen mittleren Durchmesser von etwa 50 bis 70 µm auf. Ein Abstand eines beliebigen Punktes der Oberfläche im beschichteten Teilbereich zum nächstgelegenen unbeschichteten Teilbereich ist somit kleiner ist als 35 µm. Sind die Beschichtungsinseln gleichförmig rund, so liegt der Abstand von einem beliebigen ersten Randpunkt der Oberfläche im beschichteten Teilbereich zu einen zweiten Randpunkt, der von dem ersten Randpunkt am weitesten entfernt ist, bei etwa 50 bis 70 µm.

Zur Aufbringung der Beschichtungsinseln 18 kann wie folgt vorgegangen werden:

Der Stent wird auf einen Ballon oder Katheter vormontiert. In einem Reservoir wird eine Lösung/Feinstdispersion des biodegradierbaren Polymers und des wenigstens einen Wirkstoffs bereitgestellt. Über ein ansteuerbares Mikroinjektionssystem werden anschließend Tropfen definierter Größe in ausgewählten Bereichen des Grundkörpers aufgetragen. Durch Verdampfen wird das Lösungsmittel entzogen und es bilden sich die Beschichtungsinseln definierten Durchmessers aus.

Demnach kann ausschließlich eine "ablösbare" Beschichtung als Beschichtungsinseln 18, aber auch beliebige Kombinationen von Beschichtungsinseln 18 und Körbchen 16 und/oder Kavitäten 15 und/oder zylindrisch geformten Löchern 11 und/oder konisch geformten Löchern 11a) in einem erfindungsgemäßen Stent vorliegen.

Die Anbindung der mit Wirkstoff beladenen Polymerbeschichtung am Stent kann durch Ankermoleküle erfolgen. Beispielhaft sei hier die die Verankerung mittels eines vierbindigen Siliciumatoms an der Oberfläche eines Magnesiumstents genannt, welche beispielsweise in der hier vollumfänglich beinhalteten Patentanmeldung DE 10 2006 038 231.5 der Anmelderin dargestellt ist.

Als Materialien für biodegradierbare metallische Stents sind vorzugsweise Magnesium-, Eisen- oder Wolframlegierung geeignet. Besonders bevorzugt sind Magnesiumlegierungen vom Typ WE, insbesondere WE43 geeignet. Letztere Legierung zeichnet sich durch die Anwesenheit von Seltenerden und Yttrium aus. Die genannten Materialien lassen sich einfach verarbeiten, haben geringe Materialkosten und eignen sich aufgrund der relativ raschen Degradation und des gegenüber Polymeren günstigeren elastischen Verhaltens (geringerer Recoil des Stents) besonders für die Herstellung von Stents. Zudem wurde zumindest für einen Teil der Legierungen ein positiver physiologischer Effekt der Abbauprodukte auf den Heilungsprozess festgestellt. Ferner hat es sich gezeigt, dass aus WE43 hergestellte Magnesiumstents keine störenden Magnetresonanz-Artefakte erzeugen, wie sie beispielsweise vom medizinischen Edelstahl (316A) bekannt sind, und daher kann ein Therapieerfolg mit auf Magnetresonanz basierenden Detektionsgeräten verfolgt werden. Die biodegradierbaren Metalllegierungen aus den Elementen Magnesium, Eisen oder Wolfram enthalten vorzugsweise die genannten Elemente in einem Anteil von mindestens 50 Gew%, insbesondere mindestens 70 Gew%, besonders bevorzugt mindestens 90 Gew% an der Legierung.

Erfindungsgemäße biodegradierbare metallische Stents können mit Methoden des Standes der Technik hergestellt und insbesondere beschichtet werden.

Auf die nachfolgenden Aspekte der vorliegenden Erfindung können die vorgenannten bevorzugten Ausgestaltungen jeweils angewendet werden.

Ein zweiter Aspekt der vorliegenden Erfindung betrifft ein Verfahren zur Verbesserung der Stabilität eines biodegradierbaren metallischen Stents mit folgenden Schritten:
- Bereitstellung eines biodegradierbaren metallischen Stents,
- Bereitstellung eines oder mehrerer gefäßerweiternder Wirkstoffe ausgewählt aus der Gruppe der Calciumkanalblocker, der Nitrate, der Rho-Kinase-Inhibitoren, der Endothelinrezeptor-Antagonisten, der Serotonin-Antagonisten, der Adrenozeptor-Antagonisten, der Kaliumkanalöffner, der Angiotensin-Conversion-Enzym (ACE)-Inhibitoren, der muskulotropen und/oder neurotrop-muskulotropen Spasmolytika, der Vasodilatoren mit unbekanntem Wirkmechanismus sowie der Wirkstoffe, die spasmogen wirksame Botenstoffe herunterregulieren und
- Beschichtung des biodegradierbaren metallischen Stents mit dem oder den gefäßerweiternden Wirkstoffen in einer geeigneten Matrix,
wobei der biodegradierbare metallische Stent in der Weise beschichtet wird, dass zeitnah zur Biodegradation des Stents nach Implantation der oder die gefäßerweiternden Wirkstoffe gefäßerweiternd im Bereich der Stentimplantation wirken.

Ein dritter Aspekt der vorliegenden Erfindung betrifft ein Verfahren zur Herstellung eines erfindungsgemäßen biodegradierbaren metallischen Stents mit folgenden Schritten:
- Bereitstellung eines biodegradierbaren metallischen Stents,
- Bereitstellung eines oder mehrerer gefäßerweiternder Wirkstoffe ausgewählt aus der Gruppe der Calciumkanalblocker, der Nitrate, der Rho-Kinase-Inhibitoren, der Endothelinrezeptor-Antagonisten, der Serotonin-Antagonisten, der Adrenozeptor-Antagonisten, der Kaliumkanalöffner, der Angiotensin-Conversion-Enzym (ACE)-Inhibitoren, der muskulotropen und/oder neurotrop-muskulotropen Spasmolytika, der Vasodilatoren mit unbekanntem Wirkmechanismus sowie der Wirkstoffe, die spasmogen wirksame Botenstoffe herunterregulieren und
- Beschichtung des biodegradierbaren metallischen Stents mit dem oder den gefäßerweiternden Wirkstoffen in einer geeigneten Matrix,
wobei der biodegradierbare metallische Stent in der Weise beschichtet wird, dass zeitnah zur Biodegradation des Stents nach Implantation der oder die gefäßerweiternden Wirkstoffe gefäßerweiternd im Bereich der Stentimplantation wirken.

Ein vierter Aspekt der vorliegenden Erfindung betrifft die Verwendung von einem oder mehreren gefäßerweiternden Wirkstoffen zur Verbesserung der Stabilität eines biodegradierbaren metallischen Stents, dadurch gekennzeichnet, dass
ein biodegradierbarer metallischer Stent mit einem oder mehreren gefäßerweiternden Wirkstoffen in einer geeigneten Matrix in der Weise beschichtet ist, dass zeitnah zur Biodegradation des Stents nach Implantation der oder die gefäßerweiternden Wirkstoffe gefäßerweiternd im Bereich der Stentimplantation wirken.

Ein weiterer Aspekt der vorliegenden Erfindung betrifft ein Verfahren zur therapeutischen oder prophylaktischen Behandlung von Menschen oder Tieren mit folgenden Schritten:
- Bereitstellung eines biodegradierbaren metallischen Stents,
- Bereitstellung eines oder mehrerer gefäßerweiternder Wirkstoffe ausgewählt aus der Gruppe der Calciumkanalblocker, der Nitrate, der Rho-Kinase-Inhibitoren, der Endothelinrezeptor-Antagonisten, der Serotonin-Antagonisten, der Adrenozeptor-Antagonisten, der Kaliumkanalöffner, der Angiotensin-Conversion-Enzym (ACE)-Inhibitoren, der muskulotropen und/oder neurotrop-muskulotropen Spasmolytika, der Vasodilatoren mit unbekanntem Wirkmechanismus sowie der Wirkstoffe, die spasmogen wirksame Botenstoffe herunterregulieren,
- Implantation des biodegradierbaren metallischen Stents in ein Gefäß und
- Applikationen des oder der gefäßerweiternden Wirkstoffe in einer Menge, die ausreicht, um Gefäßspasmen im Bereich der Stentimplantation der zu verhindern oder zu verringern.

Die geeigneten gefäßerweiternden Mittel können zusätzlich zusammen mit weiteren pharmazeutisch akzeptablen Hilfsstoffen, Trägern, Lösungen vorliegen.

Solche Stents werden nach den gängigen Verfahren implantiert. Stents werden dazu in einem komprimierten Zustand in das Gefäß eingeführt und dann an dem zu behandelnden Ort aufgeweitet und gegen die Gefäßwand gedrückt. Dieses Aufweiten kann beispielsweise mit Hilfe eines Ballonkatheters erfolgen. Alternativ sind auch selbstexpandierende Stents bekannt. Diese sind beispielsweise aus einem Memorymetall, wie Nitinol hergestellt.

Die Applikation, vorzugsweise eine perorale Applikation, des oder der gefäßerweiternden Wirkstoffe wird zeitnah, vorzugsweise kurz vor Stentimplantation bis zeitgleich, zur Stentimplantation begonnen und solange in einer Menge, die ausreicht einen Gefäßspasmus im Bereich der Stentimplantation zu verhindern oder zu verringern, fortgesetzt, wie der Stent biodegradiert, bevorzugt die durch die Biodegradation freigesetzte Menge an Hydroxidionen ausreicht einen Gefäßspasmus im Bereich der Stentimplantation auszulösen bzw. zu vermitteln, weiter bevorzugt innerhalb eines Zeitraumes von mindestens 4 Monaten, insbesondere bevorzugt innerhalb eines Zeitraumes von mindestens 3 bis 8 Wochen nach Stentimplantation.

Ein weiterer Aspekt der vorliegenden Erfindung betrifft ein Verfahren zur therapeutischen oder prophylaktischen Behandlung von Menschen oder Tieren mit folgenden Schritten:
- Bereitstellung eines erfindungsgemäßen biodegradierbaren metallischen Stents und
- Implantation des biodegradierbaren metallischen Stents in ein Gefäß.

In einer bevorzugten Ausführungsform wird das vorgenannte erfindungsgemäße therapeutische oder prophylaktische Verfahren zur Verbesserung der Stabilität von biodegradierbaren metallischen Stents angewendet.

Ein erfindungsgemäßer Stent wird vorzugsweise in Arterien, bevorzugt in großen Arterien implantiert.

Die Erfindung wird detailliert über die bevorzugten Ausführungsformen beschrieben. Solche Ausführungsformen stellen jedoch lediglich Beispiele dar und können vom Fachmann gegebenenfalls in äquivalenter Weise, die ebenfalls von dieser Erfindung umfasst ist, abgeändert werden.

## Patentansprüche

1. Ein biodegradierbarer metallischer Stent, der mit einem oder mehreren gefäßerweiternden Wirkstoffen ausgewählt aus der Gruppe der Calciumkanalblocker, der Nitrovasodilatatoren, der Rho-Kinase-Inhibitoren, der Endothelinrezeptor-Antagonisten, der Serotonin-Antagonisten, der Adrenozeptor-Antagonisten, der Kaliumkanalöffner, der Angiotensin-Conversion-Enzym (ACE)-Inhibitoren, der muskulotropen und/oder neurotrop-muskulotropen Spasmolytika, der Vasodilatoren mit unbekanntem Wirkmechanismus, der Wirkstoffe, die spasmogen wirksame Botenstoffe herunterregulieren, der Phosphodiesterase-5-Hemmer (PDE-5), der TRP-Hemmer oder -aktivatoren, sowie der Wirkstoffe, die TRP-Kanäle aktivieren oder inhibieren, in einer geeigneten Matrix so beschichtet ist, dass zeitnah zur Biodegradation des Stents nach Implantation der oder die gefäßerweiternden Wirkstoffe gefäßerweiternd im Bereich der Stentimplantation wirken.

2. Stent gemäß Anspruch 1, wobei die Beschichtung so ausgestaltet ist, dass die Elutionszeit des oder der gefäßerweiternden Wirkstoffe aus der Matrix [T_{E}(W)] der Degradationszeit des Stents [T_{D}(S)] entspricht.

3. Stent gemäß Anspruch 1, wobei die Beschichtung so ausgestaltet ist, dass die Elutionszeit des oder der Wirkstoffe aus der Matrix [T_{E}(W)] der Degradationszeit des Wirkstoffdepots der Matrix auf dem Stent [T_{D}(D)] entspricht und
[T_{D}(D)] und [T_{E}(W)] jeweils kleiner als die Degradationszeit des Stents [T_{D}(S)] sind
und
[T_{D}(S)] kleiner oder gleich der Elutionszeit des oder der Wirkstoffe aus der Gefäßwand [T_{E}(G)] ist.

4. Stent gemäß Anspruch 1, wobei die Beschichtung so ausgestaltet ist, dass die Degradationszeit des Stents [T_{D}(S)] kleiner als die Elutionszeit des oder der Wirkstoffe aus der Gefäßwand [T_{E}(G)] oder kleiner als die Elutionszeit des oder der Wirkstoffe aus der Matrix des Stents [T_{E}(W)] ist, wobei die [T_{E}(G)] der [T_{E}(W)] entspricht.

5. Stent gemäß einem der vorstehenden Ansprüche, wobei die Beschichtung zwei, drei oder mehrere gefäßerweiternde Wirkstoffe umfasst und die Beschichtung dergestalt ist, dass die Wirkstoffe unabhängig voneinander eluieren.

6. Stent nach einem der vorstehenden Ansprüche, wobei die Matrix zusätzlich ein oder mehrere Wirkstoffe aus der Gruppe der antiinflammatorischen und/oder antiproliferativen Wirkstoffe, Antisensenukleotide, Bisphosphonate, Antikörper, Progenitorzellen in der Matrix umfasst.

7. Stent nach einem der vorstehenden Ansprüche, wobei die Matrixbeschichtung in Kavitäten und/oder Körbchen des biodegradierbaren metallischen Stents gefüllt ist, wobei die Kavitäten gegebenenfalls zur muralen Seite hin im Durchmesser verjüngt sind.

8. Verfahren zur Verbesserung der Stabilität eines biodegradierbaren metallischen Stents mit folgenden Schritten:
- Bereitstellung eines biodegradierbaren metallischen Stents,
- Bereitstellung eines oder mehrerer gefäßerweiternder Wirkstoffe ausgewählt aus der Gruppe der Calciumkanalblocker, der Nitrovasodilatatoren, der Rho-Kinase-Inhibitoren, der Endothelinrezeptor-Antagonisten, der Serotonin-Antagonisten, der Adrenozeptor-Antagonisten, der Kaliumkanalöffner, der Angiotensin-Conversion-Enzym (ACE)-Inhibitoren, der muskulotropen und/oder neurotrop-muskulotropen Spasmolytika, der Vasodilatoren mit unbekanntem Wirkmechanismus, der der Wirkstoffe, die spasmogen wirksame Botenstoffe herunterregulieren, der Phosphodiesterase-5-Hemmer (PDE-5), der TRP-Hemmer oder - aktivatoren, sowie der Wirkstoffe, die TRP-Kanäle aktivieren oder inhibieren, und
- Beschichtung des biodegradierbaren metallischen Stents mit dem oder den gefäßerweiternden Wirkstoffen in einer geeigneten Matrix,
wobei der biodegradierbare metallische Stent in der Weise beschichtet wird, dass zeitnah zur Biodegradation des Stents nach Implantation der oder die gefäßerweiternden Wirkstoffe gefäßerweiternd im Bereich der Stentimplantation wirken.

9. Verfahren zur Herstellung eines biodegradierbaren metallischen Stents gemäß einem der Ansprüche 1 bis 5 mit folgenden Schritten:
- Bereitstellung eines biodegradierbaren metallischen Stents,
- Bereitstellung eines oder mehrerer gefäßerweiternder Wirkstoffe ausgewählt aus der Gruppe der Calciumkanalblocker, der Nitrate, der Rho-Kinase-Inhibitoren, der Endothelinrezeptor-Antagonisten, der Serotonin-Antagonisten, der Adrenozeptor-Antagonisten, der Kaliumkanalöffner, der Angiotensin-Conversion-Enzym (ACE)-Inhibitoren, der muskulotropen und/oder neurotrop-muskulotropen Spasmolytika, der Vasodilatoren mit unbekanntem Wirkmechanismus, der Wirkstoffe, die spasmogen wirksame Botenstoffe herunterregulieren, der Phosphodiesterase-5-Hemmer (PDE-5), der TRP-Hemmer oder -aktivatoren, sowie der Wirkstoffe, die TRP-Kanäle aktivieren oder inhibieren, und
- Beschichtung des biodegradierbaren metallischen Stents mit dem oder den gefäßerweiternden Wirkstoffen in einer geeigneten Matrix,
wobei der biodegradierbare metallische Stent in der Weise beschichtet wird, dass zeitnah zur Biodegradation des Stents nach Implantation der oder die gefäßerweiternden Wirkstoffe gefäßerweiternd im Bereich der Stentimplantation wirken.

10. Verwendung von einem oder mehreren gefäßerweiternden Wirkstoffen zur Verbesserung der Stabilität eines biodegradierbaren metallischen Stents, **dadurch gekennzeichnet, dass**
ein biodegradierbarer metallischer Stent mit einem oder mehreren gefäßerweiternden Wirkstoffen in einer geeigneten Matrix in der Weise beschichtet ist, dass zeitnah zur Biodegradation des Stents nach Implantation der oder die gefäßerweiternden Wirkstoffe gefäßerweiternd im Bereich der Stentimplantation wirken.
